(19) Europäisches Patentamt
European Patent Office
Office européen des brevets

(11) Publication number: 0 499 274 A1

# (12) EUROPEAN PATENT APPLICATION

(21) Application number: **92102514.4**

(51) Int. Cl.$^5$: **C07D 499/88**, A61K 31/43

(22) Date of filing: **14.02.92**

(30) Priority: **15.02.91 JP 44307/91**

(43) Date of publication of application:
**19.08.92 Bulletin 92/34**

(84) Designated Contracting States:
**AT BE CH DE DK ES FR GB GR IT LI LU MC NL PT SE**

(71) Applicant: **TAKEDA CHEMICAL INDUSTRIES, LTD.**
**1-1, Doshomachi 4-chome**
**Chuo-ku, Osaka 541(JP)**

(72) Inventor: **Maeda, Yoshiharu**
**20-38, Takabedai 1-chome**
**Tondabayashi, Osaka 584(JP)**
Inventor: **Ishibashi, Yukio**
**6-301, 6 Minamisakurazuka 3-chome**
**Toyonaka, Osaka 560(JP)**
Inventor: **Mizuno, Masahiro**
**35-20, Kotobukicho 2-chome**
**Takatsuki, Osaka 569(JP)**

(74) Representative: **Lederer, Franz, Dr. et al**
**Lederer, Keller & Riederer, Patentanwälte,**
**Lucile-Grahn-Strasse 22**
**W-8000 München 80(DE)**

## (54) Crystalline penem, its production and use.

(57) A process for producing crystalline acetoxymethyl ester of (+)-(5R,6S)-6- [(R)-1-hydroxyethyl]-3-(3-pyridyl)-7-oxo-4-thia-1-azabicyclo[3.2.0]hept-2-ene-2-carboxylic acid by adding a poor solvent to a solution of acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid in a good solvent is disclosed. The crystalline penem compound is useful as an antibacterial agent.

EP 0 499 274 A1

This invention relates to a crystalline penem compound which is useful as an antibacterial compound for medicinal use and its production.

In USP4,826,832, certain species of 2-pyridylpenem compounds are disclosed. Among them, especially the acetoxymethyl ester of (+)-(5R,6S)-6-[(R)-1-hydroxyethyl]-3-(3-pyridyl)-7-oxo-4-thia-1-azabicyclo[3.2.0]-hept-2-ene-2-carboxylic acid represented by the formula:

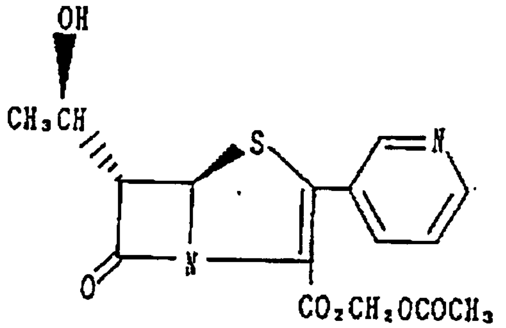

[I]

is a particulary useful penem compound which shows excellent antibacterial activity against gram-positive bacteria as well as gram-negative bacteria by oral administration, and its practical use has been studied.

However, while the penem compound [I] shows excellent antibacterial activity, it has been obtained only in the amorphous state. This amorphous solid matter is not satisfactory in stability, and it tends to undergo color change and its potency tends to decrease when it is stored for a long period of time under normal conditions, and, therefore, when it is prepared into formulations, the content of an effective component decreases. Besides, in order to make the amorphous solid matter substantially pure, complicated refining processes are required.

And, in The Journal of Antibiotics, XLIII, No.3, 306-313 (1990), the penem compound [I] has been obtained only as a foam. However, this foamy compound has also the above-mentioned defects.

For removing these defects of the above amorphous acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid, the present inventors have diligently studied to obtain penem compounds showing excellent antibacterial activity in a state in which they are stably storable. As the result, they succeeded in obtaining the penem compound [I] in a crystalline form, and found that it can be obtained as stable crystals and easily purified by crystallization. Thus the present invention has been completed.

The present invention provides crystalline acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid, having a diffraction pattern which shows main peaks at spacings of 10.0, 7.1, 5.4, 4.5, 4.24, and 4.16 Â according to powder X-ray diffraction. The said crystals can be obtained by dissolving amorphous acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid in a good solvent and then adding thereto a poor solvent which is miscible with the said good solvent, and stirring the obtained mixture. The present invention also provides a process of producing the crystals.

The amorphous acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid (this free acid will be referred to "Compound [II]" hereinafter) to be used can be obtained by subjecting the corresponding sodium salt or potassium salt to esterification with chloromethyl acetate, bromomethyl acetate or iodomethyl acetate, in accordance with the method disclosed in the above-mentioned USP 4,826,832 or The Journal of Antibiotics XLIII, No. 3, 306-313 (1990). The present inventors succeeded in obtaining seed crystals of acetoxymethyl ester of the Compound [II] by purifying the amorphous acetoxymethyl ester of the Compound [II] obtained as above by means of high performance liquid chromatography, dissolving thus purified product in ethyl ether, adding isopropyl ether to the resulting solution and then leaving the obtained mixture standing at room temperature (about 25°C) as desribed in the following Example 1. In general, compounds of this kind are considered to be difficult to obtain in crystalline form [Journal of Pharmaceutical Sciences, Vol. 66, No.9, pp. 1312-1316 (September, 1977)]. For example, as shown in the following Reference Examples 3 and 4, the present inventors attempted crystallization of the 1-(ethoxycarbonyloxy)ethyl ester, (5-methyl-2-oxo-1,3-dioxolen-4-yl)methyl ester, 1-acetoxyethyl ester, 1-(isobutoxycarbonyloxy)ethyl ester, cyclohexyloxycarbonyloxymethyl ester, 1-(isopropoxycarbonyl-oxy)ethyl ester 1-(cyclohexyloxycarbonyloxy)ethyl ester of the Compound [II] under various conditions in which the condition of this invention is involved, but no successful result was obtained.

They have found that the acetoxymethyl ester of the present invention can be specifically crystallized under specific conditions.

The present inventors have unexpectedly found that the stable crystalline penem compound [I] is produced by the first step of dissolving the said amorphous penem compound [I] into a good solvent and the second step of adding to the solution a poor solvent which is miscible with the good solvent.

The said amorphous penem compound [I] is usually dissolved in a good solvent at a temperature of -10°C to 40°C.

The good solvent to be used in the present invention is, for example usually a solvent which dissolves not less than 30 mg, preferably not less than 100 mg of the amorphous penem compound [I] per 1 ml of the solvent at room temperature (about 25°C).

Examples of said good solvent include lower alcohols such as methanol, ethanol, propanol, isopropanol and the like, esters such as ethyl acetate and the like, ethyl ether and so on. The concentration of the amorphous penem compound [I] to the good solvent is not specifically limited, but the concentration is preferably made as high as possible from the viewpoint of raising the yield of the crystals. Usually, the amount of the good solvent used is 1 to 300 ml, preferably 1 to 100 ml relative to 1 g of the amorphous penem compound [I]. Additionally, in the present invention, without isolating said amorphous penem compound from the reaction mixture after the esterification process of the penem compound (II), the reaction mixture may be diluted with said good solvent, then if desired, the resultant mixture may be purified according to the conventional means and concentrated, preferably under reduced pressure.

The poor solvent is added to the solution of the penem compound [I] in the good solvent at a temperature of -10°C to 40°C. The poor solvent to be used in the present invention is, for example usually a solvent which dissolves not more than 10mg, preferably not more than 6 mg, of the amorphous penem compound [I] per 1 ml of the solvent at room temperature (about 25°C) and at the same time is miscible with the good solvent.

Examples of said poor solvent include isopropyl ether, hexane, petroleum ether, water and the like. When an alcohol is employed as the good solvent, water or hexane may be used as the poor solvent. Usually, the amount of the poor solvent used is 1 to 300 ml, preferably 1 to 100 ml to 1 g of the amorphous penem compound [I].

A sufficient volume of a poor solvent to be added may be one being enough to cause crystallization of the penem compound [I], and the volume ratio of the good solvent to the poor solvent is about 0.1 to 10, preferably 0.4 to 10, more preferably 0.4 to 3.0.

When a mixture of a good solvent and a poor solvent which contains the penem compound [I] is stirred or left standing usually for 5 minutes to 2 hours at room temperature (-10°C to 35°C), crystallization of the penem compound [I] is usually started. In this case, crystallization may be accelerated by, for example, rubbing the wall of a vessel or the like in accordance with the conventional manner. The mixture is then keeped at 30°C or below, preferably -10°C to 15°C, and stirred or left standing for a further period ranging from 30 minutes to 20 hours to thereby complete the crystallization. The crystals thus obtained are washed with, e.g., hexane-ethanol, dried and isolated by the conventional processes.

The crystalline penem compound (I) thus obtained according to the present invention have a melting point ranging from about 107 to 108°C and show a diffraction pattern having main peaks at spacings of 10.0, 7.1, 5.4, 4.5, 4.24 and 4.16 Å according to powder X-ray diffraction.

The crystalline compound (I) is useful against various infections caused by bacteria as an antibacterial substance which shows a broad antibacterial spectrum by oral administration. For example, the crystalline compound [I] has a minimal concentration of about 8 µg/ml or less for inhibiting the growth of gram-positive and gram-negative bacteria such as Staphylococcus aureus, Staphylococcus pyogenes, Streptococcus pneumoniae, Streptococcus faecalis and bacteria of the genus Neisseria. And, the crystalline compound [I] has a minimal concentration of about 64 µg/ml or/less for inhibiting the growth of gram-negative rod bacteria such as the genus Enterobacter, Haemophilus influenza, the genus Pseudomonas and the like, and anaerobic bacteria such as the genus Bacteroides. Further, in the general infection of mice caused by Streptococcus aureus, the crystalline compound [I] shows $ED_{50}$ of about 0.5 to 15 mg/kg by oral administration.

For oral administration, the crystalline compound [I] is formulated into the form of e.g. tablets or capsules according to the conventional methods. These tablets and capsules may contain diluents such as lactose, glucose, sucrose, mannitol, sorbitol, cellulose and glycine; lubricants such as silica, talc, stearic acid or its salts and polyethylene glycol; binders such as aluminum magnesium silicate, starch, gelatine, gums, cellulose derivatives and polyvinyl pyrrolidone; disintegrators such as starch and alginic acid or its salts; and various additives such as colorants, perfumes and sweeteners and the like among others.

While the dosage may vary depending upon the subject patients (mammals including human, dog,

mouse, etc.) and their symptoms, for example, it ranges usually from about 50 mg to 1 g per day by oral administration to an adult of 70 kg weight to obtain the desired effect.

[Examples]

The following Reference Examples, Examples, Formulation Example and Experimental Example illustrate the present invention in more detail, but the present invention should not be limited to them.

The symbols used in the following examples have the following significances:

s: singlet, br: broad, d: doublet, dd: doublet of doublet, q: quartet, dq: doublet of quartet, m: multiplet, ABq: AB-type quartet, $CDCl_3$: heavy chloroform, DMSO-$d_6$: dimethylsulfoxide-$d_6$, $D_2O$: heavy water, %: weight %

In NMR (nucleic magnetic resonance spectrum), unless otherwise specified, values of chemical shifts are shown in terms of $\delta$ value (ppm) measured by using tetramethyl silane or sodium 4,4-dimethyl-4-silapentanesulfonic acid (only when heavy water is employed) as the internal standard at 60 MHz or 90 MHz.

## Reference Example 1

In 1.2 liter of tetrahydrofuran was suspended 67.3 g of potassium hydroxide. To the suspension was added 173.1 g of 2-ethyl hexanoic acid. The mixture was stirred until the potassium hydroxide was dissolved to obtain a solution of potassium 2-ethyl hexanoate.

In a mixture of 2.5 liters of methylene chloride and 1.3 liter of tetrahydrofuran was dissolved 332.4 g of allyl (+)-(5R,6S)-6-[(R)-1-hydroxyethyl]-3-(3-pyridyl)-7-oxo-4-thia-1-azabicyclo[3.2.0]hept-2-ene-2-carboxylate. To the solution were added 26.2 g of triphenyl phosphine and 11.6 g of tetrakis(triphenylphosphine)-palladium. To the mixture was added the above solution of potassium 2-ethyl hexanoate, then the mixture was stirred for 15 minutes. To the resultant mixture was added dropwise 7.5 liters of ethyl ether to cause precipitation of crystals, which was aged for 30 minutes while stirring. The crystals were collected by filtration and washed with 2.0 liters of ethyl ether. The crystals were dried under reduced pressure to give crude crystals of potassium (+)-(5R,6S)-6-[(R)-1-hydroxyethyl]-3-(3-pyridyl)-7-oxo-4-thia-1-azabicyclo[3.2.0]-hept-2-ene-2-carboxylate.

The crude crystals were dissolved in a mixture of 460 ml of methanol and 230 ml of water. To the solution was added dropwise 7.0 liters of acetonitrile to cause precipitation of crystals. The mixture was aged for one hour while cooling at 10°C or below, and the crystals were collected by filtration and washed with 3.5 liters of acetonitrile, which was followed by drying under reduced pressure to give 323.0 g of crystals of potassium (+)-(5R,6S)-6-[(R)-1-hydroxyethyl]-3-(3-pyridyl)-7-oxo-4-thia-1-azabicyclo[3.2.0]hept-2-ene-2-carboxylate.

## Reference Example 2

In 75 ml of N,N-dimethyl acetamide was dissolved 5.33 g of crystals of potassium (+)-(5R,6S)-6-[(R)-1-hydroxyethyl]-3-(3-pyridyl)-7-oxo-4-thia-1-azabicyclo[3.2.0]hept-2-ene-2-carboxylate. The solution was cooled to 5°C and there was added dropwise 3.14 g of bromomethyl acetate. The mixture was stirred for 1.5 hour at the same temperature and there were added 150 ml of ethyl acetate and 120 ml of a 5%(w/v) aqueous solution of sodium thiosulfate. The mixture was separated into the ethyl acetate layer and the aqueous layer. To the aqueous layer was added 75 ml of ethyl acetate, which was shaken for extraction. Ethyl acetate layers were combined and washed with 120 ml each portions of a saturated aqueous saline solution three times. The resultant was dried over 5 g of anhydrous magnesium sulfate, then the solvent was distilled off to leave an oily substance. The oily substance was subjected to a silica gel (150 g) column chromatography, eluting with 300 ml of a mixture of ethyl acetate and hexane (4:1) and 600 ml of ethyl acetate, successively. Fractions containing desired substance, which show a peak at UV254nm, were combined and dried over 5 g of anhydrous magnesium sulfate, followed by distilling off the solvent under reduced pressure to afford 4.21 g of acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid. NMR($CDCl_3$)$\delta$: 1.38 (3H,d, J=6Hz), 2.06 (3H, s), 3.86 (1H,dd, J=1 x 6Hz), 4.29 (1H,dq, J=6 x 6Hz), 5.6 and 5.76 (2H,ABq, J=6Hz), 5.78 (1H,d, J=1Hz), 7.2-8.8 (4H,m) ppm

This substance was in the state of hygroscopic powder and, when examined microscopically under polarized light, no birefringence nor extinction was observed even by rotating the stage using crossed nicol, thus it was confirmed to be amorphous. And, the substance was confirmed to be amorphous also by X-ray

diffraction.

Reference Example 3

About 5 mg of amorphous powder of 1-(isopropoxycarbonyloxy)ethyl ester of (+)-(5R,6S)-6-[(R)-1-hydroxyethyl]-7-oxo-3-(3-pyridyl)-4-thia-1-azabicyclo[3,2,0]hept-2-ene-2-carboxylic acid was dissolved in about 0.5 ml of ethyl ether. To the solution was added about 0.5 ml of isopropyl ether, and the mixture was left standing at 25 °C for 1 hour to give an oily substance, but no precipitation of crystals was observed.

The following esters the penem Compound [II] were subjected to the same procedure as above to attain the same result.

(1) 1-(Ethoxycarbonyloxy)ethyl ester
(2) (5-Methyl-2-oxy-1,3-dioxolen-4-yl)methyl ester
(3) 1-Acetoxyethyl ester
(4) 1-(Isobutoxycarbonyloxy)ethyl ester
(5) Cyclohexyloxycarbonyloxymethyl ester

Reference Example 4

By the same procedure as described in Example 1, 1.0g of 1-(cyclohexyloxycarbonyloxy)ethyl ester of (+)-(5R,6S)-6-[(R)-1-hydroxyethyl]-7-oxo-3-(3-pyridyl)-4-thia-1-azabicyclo[3.2.0]hept-2-ene-2-carboxylic acid was subjected to purification to obtain two types of powdery products, respectively containing only one of the two diastereoisomers of the ester (α type: 0.35g, βtype: 0.35g). About 2 mg of each of the thus purified two types of powdery products and of a mixture of these powdery products at a ratio of 1:1 was dissolved in about 0.2 ml of ethyl ether. To each of the three solutions was added about 0.2 ml of isopropyl ether, and the respective solutions were left standing at 25 °C for 1 hour only to obtain oily substances and no precipitation of crystals was observed.

Example 1

Amorphous powder (1.0 g) of acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid obtained above in Reference Example 2 was subjected to high performance liquid chromatography (Kurita System 300-W, manufactured by KURITA WATER INDUSTRIES LTD. in Japan), eluting with a mixture of 0.5%(w/v) aqueous solution of ammonium dihydrogenphosphate and acetonitrile (3:1). Fractions containing the desired compound were combined, followed by extraction with 320 ml of methylene chloride. The aqueous layer was further subjected to extraction with 160 ml of methylene chloride. The organic layers were combined and washed with 320 ml of 1% (w/v) aqueous solution of sodium hydrogencarbonate and 320 ml of saturated aqueous saline solution, successively, then dried over 50 g of anhydrous magnesium sulfate. The solvent was distilled off under reduced pressure to leave 0.84 g of the purified product of said ester in the state of powder.

About 2 mg of thus purified powdery product was dissolved in about 0.2 ml of ethyl ether at 25 °C. To the solution was added about 0.2 ml of isopropyl ether at 25 °C. The mixture was left standing at 25 °C for 1 hour to yield precipitates which were identified to be crystals through a microscope. The crystals were collected and dried to obtain white crystalline powder of the ester, m.p.107-108 °C.

Example 2

In 5.0 ml of ethyl acetate was dissolved 1.00 g of amorphous powder of acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid obtained in Reference Example 2, to which was added 2.5 ml of hexane at 25 °C. To this solution was added about 0.1 mg of crystals of the ester, as seed crystals, obtained in Example 1. The mixture was stirred at 25 °C for 1 hour, whereupon crystals precipitated out. The mixture was cooled to 5 °C and aged while stirring for one hour. The crystals were collected by filtration, washed with 10 ml of a mixture of hexane and ethyl acetate (2:1) and dried under reduced pressure to afford 0.81 g of the white crystals of said ester, m.p.107-108 °C. Powder X-ray diffraction pattern: Diffraction pattern is shown by Fig. 1 attached hereto.

Example 3

In 3 ml of ethanol was dissolved 0.61 g of amorphous powder of the ester obtained in Reference

Example 2 at 25 ° C. To this solution was added, as seed crystals, 0.1 mg of crystals of the ester obtained in Example 1. The mixture was stirred at 25 ° C for 1 hour, whereupon crystals precipitated out. To the mixture was added 2.6 ml of hexane, which was cooled at 5 ° C and aged for 4 hours, followed by filtration to collect the crystals. The crystals were washed with 15 ml of hexane and ethanol (2:1) and then dried under reduced pressure to afford 0.44 g of white crystals of the ester, m.p.107-108 ° C.
Powder X-ray diffraction pattern: Showing characteristic peak similar to that of the product obtained in Example 2.
IR (KBr method): 1772, 1714, 1322, 1186, 982 $cm^{-1}$
NMR spectra: Agreement with those observed in Reference Example 2.

Formulation Example 1

| Components | in one tablet (mg) | in 50,000 tablets (kg) |
|---|---|---|
| (1) The crystals of the penem compound [I] (the compound obtained in Example 1) | 125 | 6.250 |
| (2) Lactose | 62.3 | 3.115 |
| (3) Croscarmellose sodium | 6.4 | 0.320 |
| (4) Hydroxypropyl cellulose | 5.7 | 0.285 |
| (5) Magnesium stearate | 0.6 | 0.030 |
| Total | 200 | 10.000 |

The crystals of the penem compound [I] (6.250 kg) and lactose (3.115 kg) were mixed in a fluid-bed granulator (FD-S-2, manufactured by Pawleck in Japan) and then a 6% aqueous solution of hydroxypropyl cellulose (4.750 kg, including 0.285 kg in terms of hydroxypropyl cellulose) was sprayed to obtain granules. To the granules thus obtained were added sodium croscalmellose (0.320 kg) and magnesium stearate (0.030 kg) and the mixture was thoroughly mixed. The mixture was compressed by a rotary tabletting machine (CLEAN PRESS manufactured by KIKUSUI SEISAKUSHO LTD. in Japan) to obtain about 50,000 tablets of 8 mm diameter.

[Experimental Example]

Samples of crystalline powder of the ester obtained in Example 3 and amorphous power of the ester obtained in Reference Example 2 were respectively stored in sealed vessels at 40 ° C, 50 ° C and 60 ° C for three days under protection from light. The said samples were also stored in sealed vessels at -20 ° C as contrast. Respective residual rates of the compound (I) are shown in Table 1.

Method to measure the residual rate %

The respective residual rate (%) of the penem compound [I] was measured by High Performance Liquid Chromatography under the following conditions.

Column : Inertsill ODS-2 (4.6mm x 150mm)
Mobile Phase : 0.5% $NH_4H_2PO_4$ : $CH_3CN$ = 75:25
Detect : UV 254nm

Table 1

| Storing Conditions | Residual Rate | |
|---|---|---|
| | Amorphous Powder of Ref. Ex. 2 | Crystalline Powder of Ex. 3 |
| 40 ° C | 95.6% | 101.9% |
| 50 ° C | 83.7% | 101.7% |
| 60 ° C | 55.8% | 102.2% |

As shown in Table 1, the crystals of the penem compound [I] obtained by the present invention show excellent storability as compared with the amorphous powder thereof.

According to the present invention, acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid showing excellent antibacterial activity can be obtained as crystals showing favorable characteristic of storability. The crystals are remarkably advantageous when used for formulating them as medicinal preparations.

**Claims**

**1.** Crystalline acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid having a diffraction pattern which shows main peaks at spacings of 10.0, 7.1, 5.4, 4.5, 4.24 and 4.16 Å according to powder X-ray diffraction.

**2.** A process for producing crystalline acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid having a diffraction pattern which shows main peaks at spacings of 10.0, 7.1, 5.4, 4.5, 4.24 and 4.16 Å according to powder X-ray diffraction, which is characterized by adding to a solution of acetoxymethyl ester of (+)-(5R,6S)-6-[(R)-1-hydroxyethyl]-3-(3-pyridyl)-7-oxo-4-thia-1-azabicyclo[3.2.0]hept-2-ene-2-carboxylic acid in a good solvent a poor solvent which is miscible with the said good solvent.

**3.** A process according to claim 2, wherein the good solvent is a lower alcohol.

**4.** A process according to claim 2, wherein the good solvent is an ester.

**5.** A process according to claim 2, wherein the good solvent is ethyl ether.

**6.** A process according to claim 3, wherein the lower alcohol is methanol, ethanol, n-propanol or isopropanol.

**7.** A process according to claim 4, wherein the ester is ethyl acetate.

**8.** A process according to claim 2, wherein the poor solvent is water, iso-propyl ether, hexane or petroleum ether.

**9.** A process according to claim 2, wherein the good solvent is a lower alcohol and the poor solvent is water or hexane.

**10.** A process according to claim 2, wherein an amount of the good solvent is 1 to 300 ml relative to 1 g of acetoxymethyl ester of (+)-(5R,6S)-6-[(R)-1-hydroxyethyl]-3-(3-pyridyl)-7-oxo-4-thia-1-azabicyclo[3.2.0]-hept-2-ene-2-carboxylic acid.

**11.** A process according to claim 2, wherein an amount of the poor solvent is 1 to 300 ml relative to 1 g of acetoxymethyl ester of (+)-(5R,6S)-6-[(R)-1-hydroxyethyl]-3-(3-pyridyl)-7-oxo-4-thia-1-azabicyclo[3.2.0]-hept-2-ene-2-carboxylic acid.

**12.** A process according to claim 2, wherein a volume ratio of the good solvent to the poor solvent is 0.1 to 10.

**13.** An antibacterial composition which comprises an effective amount of the crystalline penem compound of claim 1 with a pharmaceutically acceptable carrier or carriers.

**14.** Use of the crystalline penem compound of claim 1 as a component in the preparation of an antibacterial agent.

Fig. 1

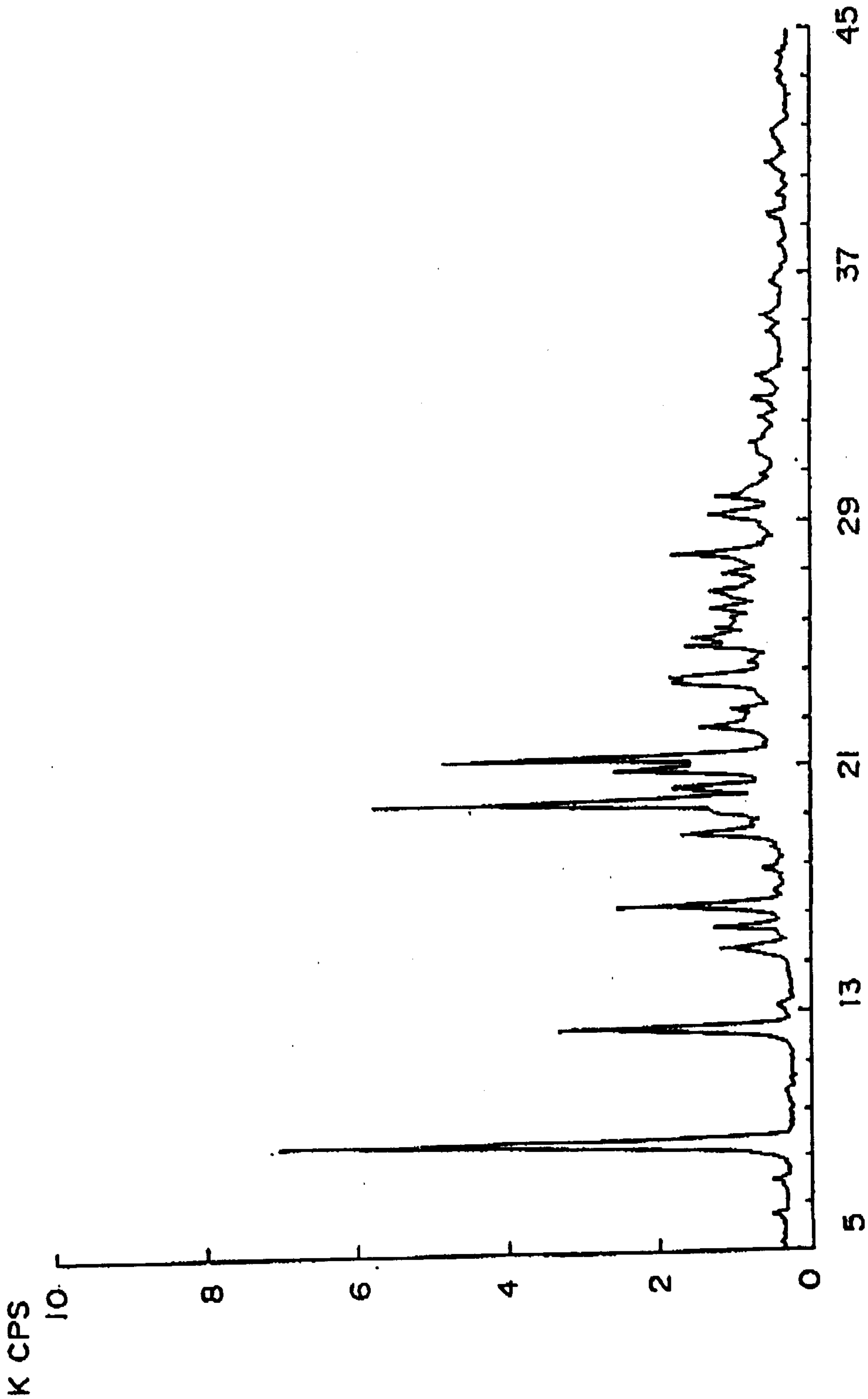

European Patent Office

# EUROPEAN SEARCH REPORT

Application Number

EP 92 10 2514

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 246 187 (CIBA-GEIGY)<br>* PAGE 35, EXAMPLE 15; CLAIMS *<br>--- | 1,13 | C07D499/88<br>A61K31/43 |
| D,A | THE JOURNAL OF ANTIBIOTICS<br>vol. 43, no. 3, 1990, TOKYO<br>pages 306 - 313;<br>A. BEDESCHI: 'SYNTHESIS AND STRUCTURE-ACTIVITY RELATIONS IN THE CLASS OF 2-(PYRIDYL)PENEMS.'<br>* The whole article, especially page 307, formula 4n and page 311, last paragraph*<br>--- | 1,13 | |
| A | CHEMICAL ABSTRACTS, vol. 97, no. 5, 2 August 1982, Columbus, Ohio, US;<br>abstract no. 38753P,<br>NOVAK, LUDVIK: 'Acetoxymethyl ester of benzylpenicillin.'<br>page 555 ;<br>* abstract *<br>& CS-A-197 138 (NOVAK) 30 April 1982<br>--- | 1,13 | |
| P,A | EP-A-0 462 521 (TAKEDA CHEMICAL INDUSTRIES, LTD. )<br>* claims *<br>----- | 1,13 | **TECHNICAL FIELDS SEARCHED (Int. Cl.5 )**<br>C07D<br>A61K |

The present search report has been drawn up for all claims

| Place of search | Date of completion of the search | Examiner |
|---|---|---|
| THE HAGUE | 28 APRIL 1992 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document